# EUROPÄISCHE PATENTSCHRIFT

(11) **EP 0 904 092 B1**
(45) Veröffentlichungstag und Bekanntmachung des Hinweises auf die Patenterteilung: **13.08.2003**
(21) Anmeldenummer: 97923083.6
(22) Anmeldetag: 15.05.1997
(51) Int. Cl.: A61K 35/78, A61P 29/00

(54) **GEREINIGTER EXTRAKT VON HARPAGOPHYTUM PROCUMBENS UND/ODER HARPAGOPHYTUM ZEYHERI DENCE, VERFAHREN ZU SEINER HERSTELLUNG UND SEINE VERWENDUNG**
PURIFIED EXTRACT OF HARPAGOPHYTUM PROCUMBENS AND/OR HARPAGOPHYTUM ZEYHERI DENCE, PROCESS FOR ITS PRODUCTION AND ITS USE
EXTRAIT PURIFIE D'HARPAGOPHYTUM PROCUMBENS ET/OU D'HARPAGOPHYTUM ZEYHERI DENCE, PROCEDE PERMETTANT DE LE PREPARER ET UTILISATION

(30) Priorität: 18.05.1996 DE 19620052
(43) Veröffentlichungstag der Anmeldung: 31.03.1999
(73) Patentinhaber: Finzelberg GmbH & Co. KG, 56626 Andernach (DE)
(72) Erfinder: BERKULIN, Willi, D-56235 Ransbach-Baumbach (DE); GAEDCKE, Frauke, D-56068 Koblenz (DE)
(74) Vertreter: Werner, Hans-Karsten, Dr.Dipl.-Chem.
(86) Internationale Anmeldenummer: EP9702491
(87) Internationale Veröffentlichungsnummer: WO97044051

(56) Entgegenhaltungen:
- EP-A- 0 524 873
- EP-B- 0 897 302
- WO-A-97/34565
- FR-A- 2 614 791
- JOURNAL DE PHARMACIE BELGE, Bd. 35, Nr. 2, März 1980 - April 1980, Seiten 143-149, XP002039420 MARYSE CAPRASSE: "DESCRIPTION, IDENTIFICATION ET USAGES TH RAPEUTIQUES DE LA "GRIFFE DU DIABLE": HARPAGOPHYTUM PROCUMBENS DC"
- DATABASE WPI Section Ch, Week 9309 Derwent Publications Ltd., London, GB; Class B04, AN 93-073947 XP002039421 & KR 9 205 686 B (DONGKUK PHARM CO) , 13.Juli 1992
- FORTH ET AL.:"Pharmakologie und Toxicologie, 6. völlig neu bearbeitete Auflage, Seiten 195-196, 320-328 und 479", 1992, Bl WISSENSCHAFTSVERLAG, MANNHEIM

## Beschreibung

Tees und Extrakte aus Harpagophytum procumbens werden seit langem in der Volksmedizin bei unterschiedlichsten Indikationen empfohlen, zum Beispiel Verdauungsstörungen verschiedener Art, rheumatischen Erkrankungen, Geburtshilfe, Wundheilung, Arterienverkalkung, allergischen Erkrankungen etc.

Über die Pflanze, die Inhaltsstoffe und deren chemische Struktur wurde ausführlich berichtet, hingegen sind pharmakologisch-toxikologische Arbeiten sehr viel spärlicher vorhanden. Eine Literaturübersicht findet sich in Erfahrungsheilkunde 2, 1995, Seiten 74 bis 79. Untersuchungen von verschiedenen Extrakten der Sekundärwurzel mit Wasser, Methanol und n-Butanol finden sich bei A. Erdös et al. in "Planta medica", Journal of Medicinal Plant Research, 1978, Vol. 34, Seiten 97 bis 108. Diese Arbeit bestätigt frühere Befunde, wonach derartige Extrakte eine mittelstarke, signifikant analgetische Wirkung zeigen, die jedoch schwächer ist als diejenige von Acetylsalicylsäure. Weiterhin kommt auch diese Arbeit zu dem Ergebnis, daß das Glykosid Harpagosid wahrscheinlich nicht allein für die hier erreichte analgetische und antiphlogistische Wirkung verantwortlich ist. Dies beweist die Wirkung von dem hochgereinigten Extrakt H 25 C₃, der 85 % Harpagosid enthält, aber in keinem der Versuchsmodelle einen bedeutend besseren Effekt zeigt als die übrigen Extrakte.

Aus Übersichten zur Eignung von Extrakten aus Radix Harpagophyti bei Behandlungen von rheumatischen Erkrankungen (Wenzel, P., Wegener, T., DAZ 135, 1131-1144) geht hervor, daß Harpagophytum-Extrakte eine mittelstarke analgetische und eine starke antiphlogistische Wirkung besitzen. Für beide Indikationsgebiete wird in aller Regel ein Wirkmechanismus beansprucht, welcher durch die Hemmung der Zyklooxygenase-Aktivität experimentell bestimmt wird (Simmet et al., Thrombosis Res. 67, 123-134, 1992).

Die Erfindung hat sich zunächst die Aufgabe gestellt, einen gereinigten und möglichst gut standardisierten Extrakt aus Harpagophytum procumbens zur Verfügung zu stellen, der nach Möglichkeit eine höhere Wirksamkeit aufweist als hochgereinigtes Harpagosid. Weiterhin hat sich die Erfindung zur Aufgabe gestellt, auch andere brauchbare Ausgangsmateriallen hierfür heranzuziehen.

Diese Aufgaben wurden jetzt überraschenderwelse gelöst durch Extrakte, welche erhältlich sind durch Auflösen von wässrigen und/oder wässrig-alkoholischen (C₁-C₃) Spissum- und/oder Trockenextrakten in mit n-Butanol gesättigtem Wasser, Extraktion mit mit Wasser gesättigtem n-Butanol, Extraktion der n-Butanolphase mit n-Butanol gesättigtem Wasser und Entfernen des n-Butanols im Vakuum. Das n-Butanol wird dabei so vollständig wie möglich entfernt. Weiterhin wurde festgestellt, dass außer Harpagophytum procumbens mit praktisch gleichem Erfolg Harpagophytum zeyheri DENCE verwendet werden kann. Es ist somit durchaus möglich, diese beiden Ausgangsdrogen alleine oder im Gemisch einzusetzen.

Es ist bis jetzt ungeklärt, wieso derartige Extrakte besser wirksam sind als die bisher bekannten und verwendeten Extrakte. Aufgrund der Literatur war nicht zu erwarten, dass ein Verfahren zum Erfolg führen würde, welches zu einer starken Anreicherung von Harpagosid führt, da dieses Glykosid für sich alleine offensichtlich nicht oder nur schwach wirksam ist. Eine bisher noch nicht bewiesene Erklärung könnte sein, dass das eigentlich wirksame Prinzip nicht das Harpagosid ist, sondern eine zumindest teilweise bei der Hochreinigung mit ihm wandernde Substanz oder Substanzgruppe. Eine weitere Möglichkeit der Erklärung besteht darin, dass es eine oder mehrere Begleitsubstanzen gibt, die synergistisch wirken, wobei dieser. Synergismus wiederum die verschiedensten Ursachen haben kann. Es könnte sich entweder um eine zweite oder weitere ebenfalls wirksame Substanzen handeln oder aber um eine oder mehrere Substanzen, die die Bioverfügbarkeit oder Stabilität des Harpagosids im Magen-Darm-Trakt erhöhen. Schließlich könnten durch das erfindungsgemäße Verfahren aus dem Vielstoffgemisch Substanzen abgereichert oder entfernt werden, die die Wirkung antagonistisch beeinflussen.

Für eine dieser Erklärungen spricht die Tatsache, daß chromatographische Untersuchungen des wäßrigen Extraktes, des wäßrigalkoholischen Extraktes, des n-Butanol-Extraktes und der erfindungsgemäßen Extrakte sich deutlich unterscheiden. Dabei liegt der Gehalt an Harpagosid erfindungsgemäß in der gleichen Größenordnung wie beim Extrakt aus reinem n-Butanol. Die Wirksamkeit des reinen n-Butanol-Extraktes liegt aber deutlich niedriger als die der erfindungsgemäßen Extrakte. Vielleicht sind Wasser oder Wasser-Alkoholgemische (C₁-C₃) in der Lage, das wirklich wirksame Prinzip oder das synergistisch wirkende Prinzip in höherer Ausbeute aus dem Pflanzenmaterial zu extrahieren. Nachdem es dann extrahiert ist, wird es auch von n-Butanol gut gelöst und somit zusammen mit dem Harpagosid angereichert.

Obwohl bisher nicht feststeht, ob Harpagosid für sich allein überhaupt wirksam ist, kann sein Gehalt dennoch zur "Standardisierung" der erfindungsgemäßen Präparation herangezogen werden. Weitere noch nicht abgeschlossene Arbeiten sollen versuchen herauszufinden, worauf die bessere antiphlogistische und analgetische Wirksamkeit der erfindungsgemäßen Extrakte beruht. Vielleicht ist es dann sogar möglich aufzuklären, ob die Wirksamkeit auf der zweiten Substanz oder Substanzgruppe allein beruht oder ein Synergismus mit Harpagosid vorhanden ist.

Für die praktische Anwendung ist es aber schon ein erheblicher Fortschritt, einen gereinigten und hochwirksamen, standardisierbaren Extrakt zur Verfügung zu stellen, welcher sowohl aus Harpagophytum procumbens als auch Harpagophytum zeyheri DENCE herstellbar ist.

Diese Extrakte können zunächst einmal in orale Applikationsformen gebracht werden, beispielsweise Tabletten, Kapseln, Säfte, Sirupe oder Lösungen.

Gegenstand der vorliegenden Erfindung sind somit nicht nur die gereinigten Extrakte, sondern auch das Verfahren zu ihrer Herstellung und ihre Verwendung zur Herstellung von Arzneimitteln zur Behandlung von Asthma bronchiale, Colitis ulcerosa, Morbus Crohn, Erkrankungen des rheumatischen Formenkreises und Indikationen, bei denen entweder durch Arzneimittel, sonstige Stoffe oder Erkrankungen eine Steigerung der Lipoxygenase besteht.

Das erfindungsgemäße Verfahren wird durch die nachstehenden Beispiele näher erläutert:

### Beispiel 1

200 kg getrocknete sekundäre Speicherwurzeln von Harpagophytum procumbens und/oder Harpagophytum zeyheri DENCE werden in einer Mühle auf eine Korngröße von kleiner 12 mm, vorzugsweise 8 bis 10 mm zerkleinert. Nach Zugabe von 2100 kg Wasser wird das Gemisch 16 h bei 70 bis 85°C erschöpfend perkoliert. Der so erhaltene Extrakt wird unter vermindertem Druck bei 150 mbar und 55°C auf einen Trockensubstanzgehalt von 65 bis 75% gebracht. Der Anteil an Harpagosid beträgt hierbei etwa 1,0 bis 1,5%.

Der so erhaltene wässrige Dickextrakt (Primärextrakt) aus ca. 180 kg wird bezüglich seines Trockenanteils mit der zehnfachen Menge an mit n-Butanol gesättigtem Wasser versetzt. Unter Rühren wird der Extrakt vollständig gelöst und mit gleichen Volumenanteilen an wassergesättigtem n-Butanol bei etwa 30°C flüssigflüssig-extrahiert. Anschließend erfolgt die Phasentrennung. Die untere wässrige Phase wird noch zwei weitere Male einer Flüssig-Flüssig-Extraktion mit jeweils dem halben Volumen an n-Butanol unterzogen.

Die von den einzelnen drei Extraktionsschritten erhaltenen Butanolphasen werden vereinigt und mit butanolgesättigtem Wasser nachgewaschen. Die vereinigte Butanolphase wird nachfolgend unter vermindertem Druck von 150 bis 200 mbar bei ca. 50 bis 60°C eingedampft, bis ein Konzentrat mit mindestens 50% Feststoffanteil vorliegt.

Der so erhaltene Dickextrakt (Sekundärextrakt) von etwa 16 kg wird auf einem Sprühtrocknungsaggregat bei 60 bis 80°C nativ getrocknet. Dabei wird das n-Butanol praktisch vollständig entfernt. Der resultierende Trockenextrakt wird gemahlen. Der erfindungsgemäße Trockenextrakt enthält einen Harpagosid-Gehalt nach HPLC von ca. 15 bis 30% und weist ein Droge-Extrakt-Verhältnis (DEV) von 25 bis 35 : 1 auf.

### Beispiel 2 (Vergleichsbeispiel)

Der gemäß Beispiel 1 erhaltene wässrige Dickextrakt (Primärextrakt) aus ca. 180 kg wird im Sprühtrocknungsverfahren bei ca. 80°C getrocknet. Durch 30 min Behandlung in einem Vertikalmischer und nachfolgende Feinstmahlung < 1mm wird ein homogener Trockenextrakt (Primärextrakt) hergestellt. Dieser getrocknete wäßrige Extrakt von Harpagophytum procumbens und/oder Harpagophytum zeyheri DENCE wird mit etwa der zehnfachen Menge Wasser gelöst, welches mit n-Butanol gesättigt ist (Ausgangslösung). Diese Ausgangslösung wird filtriert und mit dem gleichen Volumen an mit Wasser gesättigtem n-Butanol ausgeschüttelt. Es erfolgt Phasentrennung. Die obere Butanol-Phase wird mit mit Butanol gesättigtem Wasser nachgewaschen (Verhältnis 1:1 V/V). Die untere Wasser-Phase wird weitere zweimal, somit insgesamt dreimal mit mit Wasser gesättigtem Butanol extrahiert. Die vereinigten Butanol-Phasen werden im Vakuum (200 mbar, 60°C) zur Trockne eingeengt. Auch die wäßrige Phase wird vereinigt und eingeengt. Die vergleichende Untersuchung der Ausgangslösung, der Wasser-Phase und der Butanol-Phase ergab folgende Ergebnisse:

| Parameter | Ausgangslösung | Wasser-Phase | Butanol-Phase |
|---|---|---|---|
| Harpagosidgehalt HPLC [%] | 0,21 | n.n.w. | 2,25 |
| Trockensubstanzgehalt (TS) [%] | 8,9 | 2,9 | 12,0 |
| berechneter Harpagosidgehalt in TS m/m [%] | 2,36 | - | 18,75 |
| nativer Extrakt Menge [g] | 17,80 | 15,72 | 2,02 |
| Extraktionsstoff-Ausbeute [%] | - | 88,4 | 11,4 |
| Harpagosid-Menge [g] | 0,42 | - | 0,38 - |
| Harpagosid-Ausbeute [%] | - | - | 90,5 |
| DEV ₙₐₜᵢᵥ | 1,8:1 | - | 15,8:1 |

Aus dieser Tabelle ergibt sich, daß die Flüssig-Flüssig-Extraktion mit mit Wasser gesättigtem Butanol zu einer selektiven Anreicherung von Harpagosid führt und man dabei zu ähnlichen Konzentrationen kommt wie bei einer reinen Butanol-Extraktion.

Beim erfindungsgemäßen Verfahren verbleiben aber die überwiegenden Mengen der Trockensubstanz und der darin enthaltenen Sekundärstoffe wie Zucker in der wäßrigen Phase, während Harpagosid, andere Iridoidverbindungen und weitere wirksame Bestandteile in die n-Butanol-Phase übergehen.

### Beispiel 3

200 kg getrocknete sekundäre Speicherwurzeln von Harpagophytum procumbens DE CANDOLLE und/oder Harpagophytum zeyheri DENCE werden in einer Mühle auf eine Korngröße von 8 bis 10 mm zerkleinert. Nach Zugabe von ca. 3600 kg Ethanol 90% (V/V) wird das Gemisch 16 h bei 20 bis 30°C erschöpfend perkoliert. Der so erhaltene Extrakt wird unter vermindertem Druck von 150 mbar bei 55°C auf einen Trockensubstanzgehalt von < 95% gebracht. Es resultieren 20 bis 30 kg Trockenextrakt mit einem DEV von 6 bis 12 : 1. Der Anteil an Harpagosid beträgt hierbei etwa 1 bis 1,5%. Der so eingesetzte Extr. Harpagophytum e rad. spir. sicc. aus ca. 180 kg wird wie in Beispiel 1 beschrieben weiter aufgearbeitet.

Der Harpagosidgehalt beträgt 11,6 m/m [%], die native Extraktmenge 0,9 g und die Extraktionsstoff-Ausbeute 8,6%. Die Menge an Harpagosid beträgt 0,112 g. Die Ausbeute beträgt 93,4%. Das Droge-Extraktions-Verhältnis DEVₙₐₜᵢᵥ ist in der Ausgangslösung 3:1 und in der Butanolphase 32:1.

Auch dieser Extrakt enthält somit stark angereichert Harpagosid. Das HPLC-Chromatogramm des so erhaltenen Extraktes ist dem des Extraktes gemäß Beispielen 1 und 2 sehr ähnlich.

Vergleichende pharmakologische Untersuchungen durch die Professoren Simmet und Loew mit einem nur wäßrigen Extrakt, einem nur n-Butanol-Extrakt und einem erfindungsgemäßen Extrakt ergaben eine deutlich bessere Wirksamkeit der erfindungsgemäßen Extrakte gegenüber dem reinen n-Butanol-Extrakt, welcher eine vergleichbar hohe Konzentration an Harpagosid enthält.

Untersucht wurden die folgenden Extrakte:

| Nr. des Extraktes | Extraktions-mittel | DEV ₙₐₜᵢᵥ | Harpagosid-gehalt [%] |
|---|---|---|---|
| 1 | Wasser | 2 : 1 | 2,4 |
| 4 | 100% wasser-gesättigtes n-Butanol | 10 : 1 | 11,6 |
| 6 | n-Butanol-Extrakt von 1 (Beispiel 2) | 15,8 : 1 | 18,8 |

Untersucht wurden der Einfluß der Extrakte auf die Cysteinyl-LT-Biosynthese und die Thromboxan B₂-Biosynthese. Dazu wurden je 2 ml Vollblut von gesunden, männlichen Probanden (20 bis 25 Jahre) mit Heparin 10 U/ml (Endkonzentration) antikoaguliert und mit dem jeweiligen Testextrakt für 15 min bei 37°C in Polystyrolröhrchen vorinkubiert. Nach diesem Zeitraum wurde Ionophor A23187 10 µM (Endkonzentration) zugesetzt und die Inkubation bei 37°C für weitere 60 min fortgeführt. Nach Zentrifugation wurde das Plasma abgenommen, ein Aliquot asserviert und eine Proteinfällung mit vorgekühltem Aceton vorgenommen (-20°C, 30 min). Die Proben wurden sodann unter Unterdruck am Rotavapor eingeengt und in 10 mM Trispuffer, pH 7,4 resuspendiert. Dieses Material wurde zur radioimmunologischen Analyse der Cysteinyl-Leukotriene (LT) verwendet. Die anti-Cysteinyl-LT-Antikörper erkennen hauptsächlich LTC₄, weisen aber je 70% Kreuzreaktion mit LTD₄ und LTE₄ auf. Die Standardkurven wurden mit LTC₄ erstellt. Thromboxan (TX) B₂ wurde in Aliquots der Plasmaproben direkt radioimmunolgisch bestimmt.

Hieraus ergibt sich, daß die Wirkungen auf die Thromboxan B₂-Biosynthese vergleichbar sind, während die Wirkungen auf die Cysteinyl-LT-Biosynthese sich deutlich unterscheiden, und daß der erfindungsgemäße Extrakt Nr. 6 deutlich stärker und selektiver wirkt.

Daraus ergibt sich, daß der erfindungsgemäße Extrakt einsetzbar ist bei den Indikationen Asthma bronchiale, Colitis ulcerosa, Morbus Crohn, Erkrankungen des rheumatischen Formenkreises und Indikationen, bei denen entweder durch Arzneimittel, sonstige Stoffe oder Erkrankungen eine Steigerung der Lipoxygenase besteht.

Weitere pharmakologische Untersuchungen mit einem Extrakt mit 100% Methanol (Extrakt Nr. 5) zeigten hingegen auf die Cysteinyl-LT-Biosynthese keine signifikante Wirkung, während der Extrakt gemäß Beispiel 3 (Extrakt Nr. 8) ähnlich gute Wirkung zeigte wie Extrakt Nr. 6 entsprechend Beispiel 2.

Untersuchungen mit künstlichem Magensaft und künstlichem Darmsaft haben gezeigt, daß Harpagosid sowohl in reiner Form wie in verdünnter Form mindestens zwei Stunden stabil ist. Pharmkokinetische Untersuchungen haben gezeigt, daß bei Applikationsmengen zwischen 200 und 800 mg des Extraktes gemäß Beispiel 2 die höchsten Blutspiegel im Zeitraum von 1 bis 4 Stunden nach Einnahme beobachtet werden.

## Patentansprüche

1. Gereinigter Extrakt von Harpagophytum procumbens und/oder Harpagophytum zeyheri DENCE erhältlich durch Auflösung von wässrigen und/oder wässrig-alkoholischen (C₁-C₃) Spissum- und/oder Trockenextrakten in mit n-Butanol gesättigtem Wasser, Extraktion mit mit Wasser gesättigtem n-Butanol, Extraktion der n-Butanolphase mit n-Butanol gesättigtem Wasser und Entfernen des n-Butanols aus der n-Butanolphase im Vakuum.

2. Verfahren zur Herstellung von gereinigtem Extrakt von Harpagophytum procumbens und/oder Harpagophytum zeyheri DENCE, **dadurch gekennzeichnet, dass** die wässrigen und/oder wässrig-alkoholischen Spissum- und/oder Trockenextrakte in mit n-Butanol gesättigtem Wasser gelöst werden und diese Lösung mit mit Wasser gesättigtem n-Butanol extrahiert wird, danach die n-Butanolphase mit n-Butanol gesättigtem Wasser extrahiert wird, woraufhin das n-Butanol aus der n-Butanolphase im Vakuum entfernt wird.

3. Verwendung des gereinigten Extraktes von Harpagophytum procumbens und/oder Harpagophytum zeyheri DENCE gemäß Anspruch 1 zur Herstellung von Arzneimitteln zur Behandlung von Asthma bronchiale, Colitis ulcerosa, Morbus Crohn, Erkrankungen des rheumatischen Formenkreises und Indikationen, bei denen entweder durch Arzneimittel, sonstige Stoffe oder Erkrankungen eine Steigerung der Lipoxygenase besteht.

## Claims

1. A purified extract from *Harpagophytum procumbens* and/or *Harpagophytum zeyheri* DENCE, obtainable by dissolving aqueous and/or aqueous/alcoholic (C₁-C₃) pillular and/or dry extracts in water saturated with n-butanol, extracting with n-butanol saturated with water, extracting the n-butanol phase with water saturated with n-butanol, and removing the n-butanol from the n-butanol phase in vacuo.

2. A process for the preparation of a purified extract from *Harpagophytum procumbens* and/or *Harpagophytum zeyheri* DENCE, **characterized by** dissolving the aqueous and/or aqueous/alcoholic pillular and/or dry extracts in water saturated with n-butanol and extracting the resulting solution with n-butanol saturated with water, then extracting the n-butanol phase with water saturated with n-butanol, followed by removing the n-butanol from the n-butanol phase in vacuo.

3. Use of the purified extract from *Harpagophytum procumbens* and/or *Harpagophytum zeyheri* DENCE according to claim 1 for the preparation of medicaments for the treatment of asthma bronchiale, colitis uicerosa, Crohn's disease, rheumatic diseases and indications in which there is an increase in lipoxygenase level due to medicaments, other substances or diseases.

## Revendications

1. Extrait purifié d'Harpagophytum procumbens et/ou d'Harpagophytum zeyheri DENCE obtenu par dissolution d'extraits concentrés (« spissum extrakten ») aqueux et/ou aqueux/alcooliques (en C₁-C₃) et/ou d'extraits secs dans de l'eau saturée avec du n-butanol, extraction par du n-butanol saturé en eau, extraction de la phase n-butanol avec de l'eau saturée en n-butanol et élimination du n-butanol de la phase n-butanol sous vide.

2. Procédé de préparation d'extrait purifié d'Harpagophytum procumbens et/ou d'Harpagophytum zeyheri DENCE, **caractérisé en ce qu'**on dissous les extraits concentrés aqueux et/ou aqueux/alcooliques (en C₁-C₃) et/ou les extraits secs dans de l'eau saturée en n-butanol, qu'on extrait cette solution avec du n-butanol saturé en eau, qu'on extrait ensuite la phase n-butanol avec de l'eau saturée en n-butanol, après quoi on enlève le n-butanol de la phase n-butanol sous vide.

3. Utilisation de l'extrait purifié d'Harpagophytum procumbens et/ou d'Harpagophytum zeyheri DENCE selon la revendication 1 pour préparer des médicaments destinés au traitement de l'asthme bronchique, la colite ulcéreuse, la maladie de Crohn, des maladies rhumatismales, et des indications d'augmentation de la lipoxygénase par des médicaments, d'autres substances ou des maladies.
